Europäisches Patentamt

European Patent Office

Office européen des brevets

(11) Veröffentlichungsnummer: **0 483 597 A2**

# EUROPÄISCHE PATENTANMELDUNG

(21) Anmeldenummer: **91117619.6**

(22) Anmeldetag: **16.10.91**

(51) Int. Cl.5: **C12P  17/12**, //(C12P17/12, C12R1:645)

(30) Priorität: **27.10.90 DE 4034218**

(43) Veröffentlichungstag der Anmeldung:
**06.05.92 Patentblatt  92/19**

(84) Benannte Vertragsstaaten:
**AT BE CH DE DK ES FR GB GR IT LI LU NL SE**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Schwartz, Harry, Dr.**
**Zaunkönigweg 18**
**W-6138 Hofheim-Diedenbergen(DE)**
Erfinder: **Böttcher, Henning, Dr.**
**Soderstrasse 95**
**W-6100 Darmstadt(DE)**

(54) **Verfahren zur Herstellung von Carebastin.**

(57) Die Erfindung betrifft ein neues Verfahren zur Herstellung von Carebastin aus Ebastin durch Oxidation mit Hilfe von Mikroorganismen. Carebastin dient zur Behandlung allergischer Erkrankungen und stellt ein Antihistaminikum dar.

EP 0 483 597 A2

Die Erfindung betrifft ein neues Verfahren zur Herstellung von Carebastin, dadurch gekennzeichnet, daß man Ebastin mit einem Mikroorganismus oxidiert.

Die chemische Herstellung von Carebastin ist aus EP 134 124 bekannt, jedoch wird bei dieser Methode ein zugrundeliegender Ester verseift.

Der Erfindung lag die Aufgabe zugrunde, ein verbessertes Verfahren zur Herstellung des pharmazeutisch wertvollen Carebastins zu entwickeln.

Es wurde gefunden, daß Ebastin überraschenderweise von Mikroorganismen selektiv zu Carebastin oxidiert wird. Dieses neue Verfahren hat gegenüber dem bekannten Verfahren den Vorteil, daß es einen direkten geradlinigen Zugang zu Carebastin, unter Umgehung mehrstufiger chemischer Synthesen mit weitaus geringerer Selektivität, liefert. Zusätzlich ist die neue Methode leicht auf technische Maßstäbe übertragbar und bietet daher gegenüber dem herkömmlichen Verfahren den Vorteil, daß Carebastin damit erstmals in größeren Mengen und in guter Gesamtausbeute zugänglich ist.

Das Verfahren eignet sich ebenso zur Oxidation ähnlicher Verbindungen, insbesondere zur Oxidation von Terfenadin.

Carebastin kann als Arzneimittelwirkstoff in der Human- und Veterinärmedizin, insbesondere als Antiallergikum und zur Behandlung von cardiovaskulären Erkrankungen verwendet werden.

Im einzelnen wird Ebastin zweckmäßig direkt oder in Lösung, vorzugsweise in Dimethylformamid (DMF), aber auch in Dimethylsulfoxid (DMSO), Dimethylacetamid (DMA), Dimethoxyethan (DME), Tetrahydrofuran (THF) sowie Dibutyl-, Diisopropyl-, Diethylformamid, 1-Methyl-, 1-Ethyl-, 1-Cyclohexylpyrrolidon, 4-Formylmorpholin, 1-Formylpiperidin, 1-Formylpyrrolidin, Tetramethyl-, Tetraethyl-, Tetrabutylharnstoff, Tripiperidino-, Tripyrrolidinophosphinoxid, Sulfolan oder N-Methylcaprolactam oder Mischungen der genannten Lösungsmittel, bei einem pH-Wert zwischen 3 und 9, insbesondere aber im Neutralbereich, zu einer Kulturlösung der Mikroorganismen, die nach an sich bekannten Verfahren bereitet wird, zugegeben und 2-200 Stunden, bevorzugt 1 Woche, bei einer Temperatur zwischen etwa 10 und 60 °C, zweckmäßig 25-35 °C, inkubiert.

Gegenstand der Erfindung ist ferner die Verwendung von Mikroorganismen, vorzugsweise von filamentösen Pilzen, bevorzugt der Gattung Cunninghamella, insbesondere der Art Cunninghamella elegans, aber auch von Cunninghamella blakesleeana, zur chemoselektiven Oxidation von Ebastin.

Beispiel 1

0,5 1 Kulturlösung (pH-Wert 7.0) werden mit Cunninghamella elegans (DSM 1908) beimpft. Nach dreitägiger Kultur bei 28 °C werden 50 mg Ebastin, gelöst in 0,5 ml DMF, zugesetzt.

Nach weiterer Inkubation von 5 Tagen bei 28 °C wird die vorliegende Suspension mit HCl angesäuert und mit Dichlormethan extrahiert. Nach Trocknung der organischen Phase über-Natriumsulfat wird das Lösungsmittel entfernt. Das als Rückstand erhaltene Carebastin wird mittels präparativer DC oder säulenchromatographisch (Dichlormethan/Methanol 10 : 1; Kieselgel) gereinigt; F. 93-95 °C.

Beispiel 2

Man kultiviert Cunninghamella blakesleeana (ATCC 8688a) in 1 l Kulturlösung bei einem pH-Wert von 7. Der Kultur fügt man 0,5 g Ebastin, gelöst in 5 ml DMF, hinzu. Die Inkubation erfolgt bei 30 °C über 1 Woche hinweg. Aufarbeitung analog Beispiel 1 ergibt Carebastin; F. 93-95 °C.

Beispiel 3

Zu 1 l Kulturlösung von Cunninghamella elegans (DSM 1908) wird bei pH 6 0,2 g Ebastin gelöst in 2 ml DMSO zugegeben. Nach einer Inkubation von 4 Tagen bei 30 °C arbeitet man analog Beispiel 1 auf und erhält Carebastin; F. 93-95 °C.

Beispiel 4

Analog Beispiel 1 führt man den Versuch in einem 100 1 Reaktor aus, indem man zu 100 1 Kulturlösung von Cunninghamella elegans (DSM 1908) 5 g Ebastin in 50 ml DMF gelöst hinzufügt. Aufarbeitung gemäß Beispiel 1 liefert Carebastin; F. 93-95 °C.

Beispiel 5

Cunninghamella blakesleeana (ATCC 8688a) wird in 1 l Kulturlösung 3 Tage kultiviert. Die gut gewachsenen Zellen werden vom Nährmedium separiert, 2 mal mit Phosphatpuffer (0.1 M; pH 7.2) gewaschen, im gleichen Puffer resuspendiert. Der Zellsuspension werden 0,1 % Ebastin zugesetzt. Nach Inkubation für 7 Tage bei 28 °C erfolgt die Aufarbeitung analog Beispiel I. Man erhält Carebastin; F. 93-95 °C.

Beispiel 6

Analog Beispiel 5 wird anstelle von Cunninghamella blakesleeana (ATCC 8688a) Cunninghamella elegans (DSM 1908) eingesetzt. Kultur, Inkubation und Aufarbeitung entsprechend Beispiel 5 liefert Carebastin; F. 93-95 °C.

**Patentansprüche**

1.  Verfahren zur Herstellung von Carebastin, dadurch gekennzeichnet, daß man Ebastin mit einem Mikroorganismus oxidiert.

2.  Verwendung von Mikroorganismen zur chemoselektiven Oxidation von Ebastin zu Carebastin.